# EUROPEAN PATENT APPLICATION

(11) **EP 4 123 657 A1**
(43) Date of publication of application: **25.01.2023**
(21) Application number: 22194355.8
(22) Date of filing: 04.12.2013
(51) Int. Cl.: G16H 10/60

(54) **PRESENTATION OF PHYSIOLOGICAL DATA**

(62) Divisional of application: 13811085.3
(71) Applicant: Apple Inc., Cupertino CA 95014 (US)
(72) Inventor: Soli, Christopher D., Cupertino 95014 (US); Davydov, Anton M., Cupertino 95014 (US); Kennedy, Zachery, Cupertino 95014 (US); Minjack, Zachery, Cupertino 95014 (US); Park, Dennis S., Cupertino 95014 (US); Edwards, Dylan R., Cupertino 95014 (US); Beberg, Adam L., San Jose 95129 (US); Yang, Lawrence Y., Cupertino 95014 (US); Eun, Christine, San Francisco 94117 (US); Greer, Stephanie, Cupertino 95014 (US); Keen, Daniel S., Cupertino 95014 (US); Lemay, Stephen O., Cupertino 95014 (US); Lynch, Kevin, Cupertino 95014 (US); Maric, Natalia, Cupertino 95014 (US); Nag, Divya, Cupertino 95014 (US); Novick, Gregory, Cupertino 95014 (US); O'Reilly, Michael, Cupertino 95014 (US); Pahwa, Aroon, Cupertino 95014 (US); Pitschel, Donald W., Cupertino 95014 (US); Mistri, Afshad M., Cupertino 95014 (US); Tickner, Simon, Cupertino 95014 (US); Weber, Rebecca L., Cupertino 95014 (US); Blahnik, Jay J., California 95014 (US); Foster, James H., California 95014 (US); Lysik, Stacey, California 95014 (US)
(74) Representative: Barton, Russell Glen

(57) **Abstract**

The present disclosure relates to aggregating and sharing wellness data. The wellness data can be received by a user device from any number of sensors external or internal to the user device, from a user manually entering the wellness data, or from other users or entities. The user device can securely store the wellness data on the user device and transmit the wellness data to be stored on a remote database. A user of the device can share some or all of the wellness data with friends, relatives, caregivers, healthcare providers, or the like. The user device can further display a user's wellness data in an aggregated view of different types of wellness data. Wellness data of other users can also be viewed if authorizations from those users have been received.

## Description

### Field

The following disclosure relates generally to data management and, more specifically, to aggregating and sharing wellness data.

### Background

Approximately 133 million Americans currently suffer from at least one chronic condition. This number is expected to rise to approximately 165 million by the year 2020. As a result, the cost of healthcare in the United States is expected to increase dramatically. Attempts have been made to improve the health of individuals by providing them with tools to monitor and track their wellness data. Wellness data can generally include any type of data associated with a person's health, such as their weight, heart rate, blood pressure, blood glucose level, medication compliance, activity level, or the like. Users can monitor their wellness using devices, such as blood pressure cuffs, blood glucose monitors, electrocardiograms, step counters, and the like. Software applications (e.g., Apps) associated with each of these devices have also been developed to allow users to track their wellness data over time. While each application can be used to view useful information about a user's health, current applications are limited in their ability to allow users to store, view, and share wellness data collected by different devices.

### Summary

The present disclosure relates to processes for aggregating and sharing wellness data. One example process can include causing a display of an aggregated view of a plurality of types of wellness data, wherein the aggregated view comprises a plurality of partitions, each partition of the plurality of partitions associated with a type of the plurality of types of wellness data; receiving a selection of a partition of the plurality of partitions; and causing a display of an expanded view of the selected partition of the plurality of partitions.

In one example, in the aggregated view, each partition of the plurality of partitions comprises an identifier of an associated type of wellness data and a first portion of the associated type of wellness data displayed therewith, and wherein the expanded view comprises a larger view of the selected partition of the plurality of partitions and a second portion of the associated type of wellness data displayed therewith. In another example, the first portion of the wellness data comprises a most recent value of the associated type of wellness data, and wherein the second portion of the associated type of wellness data comprises a representation of the associated type of wellness data over time.

In one example, causing a display of the expanded view of the selected partition comprises causing a display of a collapsed set of partitions representing partitions of the plurality of partitions that were not selected. In another example, the plurality of partitions are ordered within the display based on a frequency of use of an associated type of wellness data, a time of most recently added value of the associated type of wellness data, or a time of day.

In one example, the expanded view of the selected partition comprises a selectable element to share the wellness data associated with the partition through email or text message.

In one example, the plurality of types of wellness data comprises weight data, blood sugar data, blood pressure data, activity data, or heart rate data. In another example, at least one of the plurality of types of wellness data is generated from sensor data obtained from a plurality of sensors.

In one example, the expanded view comprises a graph of the associated type of wellness data, and wherein the graph comprises segments generated from wellness data obtained from different sensors.

In one example, a partition of the plurality of partitions is a user-generated partition. In another example, the plurality of partitions have the appearance of a plurality of cards displayed in a stack.

Another example process can include causing, on a device, a display of a plurality of partitions, wherein each partition of the plurality of partitions is associated with a type of wellness data of a plurality of types of wellness data; in response to detecting a change in an orientation of the device, selecting a subset of the plurality of partitions; and causing a display of the selected subset of the plurality of partitions.

In one example, the change in orientation of the device is detected based on data from at least one of a gyroscope of the device, an accelerometer of the device, or a combination thereof. In another example, detecting the change in the orientation of the device comprises detecting a threshold amount of change in the orientation of the device.

In one example, each of the displayed subset of the plurality of partitions comprises a graph representation of at least a portion of the associated type of wellness data displayed therewith. In another example, selecting a subset of the plurality of partitions comprises: identifying correlations between the plurality of types of wellness data; and selecting partitions associated with correlated types of wellness data as the subset of the plurality of partitions.

Another example process can include causing a display of a first plurality of partitions associated with a first user, wherein each partition of the first plurality of partitions is associated with a type of wellness data of the first user; and in response to receiving a request to view a second plurality of partitions associated with a second user, causing a display of a second plurality of partitions associated with a second user, wherein each partition of the second plurality of partitions is associated with a type of wellness data of the second user.

In one example, the request to view the second plurality of partitions comprises a request to scroll the displayed first plurality of partitions in a lateral direction. In another example, the request to view the second plurality of partitions comprises a selection of the second user from a list of users.

In one example, the first user has been authorized by the second user to view the second plurality of partitions.

Another example process can include receiving an identification of a user authorized to access a set of wellness data; in response to detecting an update to the set of wellness data, transmitting a notification to the user authorized to access the set of wellness data notifying the user authorized to access the set of wellness data that the update to the set of wellness data has been detected; and transmitting at least a portion of the set of wellness data to the user authorized to access the set of wellness data.

In one example, the identification of the user authorized to access the set of wellness data comprises a name, a username, or contact information. In another example, the at least a portion of the set of wellness data is transmitted in response to receiving a request from the user authorized to access the set of wellness data.

Another example process can include receiving, from a first user, a request to access wellness data associated with a second user; transmitting, to the second user, a request to authorize the first user to access the wellness data associated with the second user; and in response to receiving an authorization from the second user, transmitting the wellness data associated with the second user to the first user.

In one example, the first user is a health care provider and the second user is a patient, and wherein the request to access the wellness data associated with the second user is received prior to an appointment between the first user and the second user. In another example, the request to authorize the first user to access the wellness data associated with the second user is displayed on a mobile device of the second user.

Systems and non-transitory computer-readable storage media for performing these processes are also provided.

### Brief Description of the Drawings

FIG. 1A illustrates a block diagram of an example system for aggregating wellness data according to various examples.
FIG. 1B illustrates a block diagram of an example system for sharing wellness data according to various examples.
FIG. 2 illustrates an example process for authorizing and pushing wellness data to authorized other users according to various examples.
FIG. 3 illustrates an example process for authorizing users to pull wellness data according to various examples.
FIGs. 4-9 illustrate example interfaces for displaying aggregated wellness data according to various examples.
FIG. 10 illustrates an example process for displaying aggregated wellness data according to various examples.
FIG. 11 illustrates an example interface for displaying aggregated wellness data according to various examples.
FIG. 12 illustrates an example process for displaying aggregated wellness data according to various examples.
FIG. 13 illustrates an example interface for displaying aggregated wellness data of other users according to various examples.
FIG. 14 illustrates another example interface for displaying aggregated wellness data of other users according to various examples.
FIG. 15 illustrates an example process for displaying aggregated wellness data of other users according to various examples.
FIG. 16 illustrates an example computing system for aggregating and sharing wellness data according to various examples.

### Detailed Description

In the following description of the disclosure and examples, reference is made to the accompanying drawings in which it is shown by way of illustration specific examples that can be practiced. It is to be understood that other examples can be practiced and structural changes can be made without departing from the scope of the disclosure.

The present disclosure relates to aggregating and sharing wellness data. The wellness data can be received by a user device from any number of sensors external or internal to the user device, from a user manually entering the wellness data, or from other users or entities. The user device can securely store the wellness data on the user device and transmit the wellness data to be stored on a remote database. A user of the device can share some or all of the wellness data with friends, relatives, caregivers, healthcare providers, or the like. The user device can further display a user's wellness data in an aggregated view of different types of wellness data. For example, the aggregated view can include a set of partitions, where each partition corresponds to a different type of wellness data. Wellness data of other users can also be viewed if authorizations from those users have been received. In some examples, the partitions can be displayed as having the appearance and associated animations of a stack of cards, where each card corresponds to a different partition (and thus, a different type of wellness data). In this view of stacked cards, each card can display a partial view of a portion of its corresponding wellness data. When the user selects one of the cards, a first expanded view of the selected card including at least one of first reconfigured data, additional data, or an expanded view of the original data can be displayed. A second expanded view of the selected card can be displayed in response to a change in the orientation of the user device. The second expanded view can include at least one of second reconfigured data, additional data, or an expanded view of the original data can be displayed. In one example, the second expanded view can include a graph of the wellness data over time.

### SYSTEM OVERVIEW

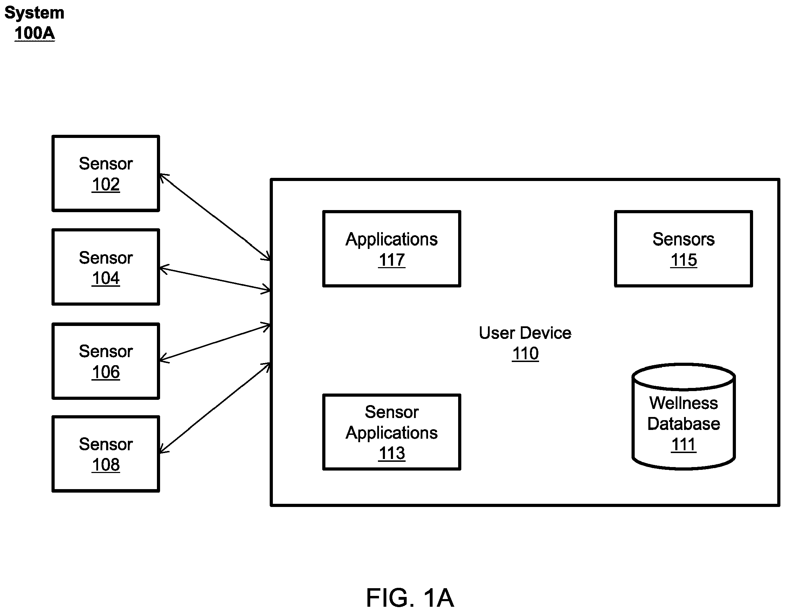

FIG. 1A illustrates an example system 100A for aggregating and sharing wellness data. As mentioned above, wellness data can include, but is not limited to, any type of data associated with a person's health, such as their weight, heart rate, blood pressure, blood glucose level, medication compliance, activity level, or the like. System 100A can be used to collect wellness data associated with a user, store the wellness data, present the wellness data to the user in useful ways, and selectively share the user's wellness data with other users or entities based on permissions set by the user. In addition, in some examples, system 100A can further be used to collect non-wellness data along with wellness data, correlate the non-wellness data with the wellness data, and display the non-wellness data with the wellness data.

System 100A can include one or more user devices 110 including any electronic device, such as a mobile phone, tablet computer, desktop computer, laptop computer, PDA, or the like. User device 110 can include an operating system and a wellness database 111 for securely storing wellness or non-wellness data along with associated metadata, such as the time the data was recorded, type of data, device used to record the data, user associated with the data, and the like. User device 110 can further include application programming interfaces (APIs) with access controls for storing data in the wellness database 111 and for accessing data stored in the wellness database 111.

User device 110 can be configured to receive wellness or non-wellness data from various sources and can store the received data in the wellness database 111. For example, user device 110 can be configured to receive wellness or non-wellness data from sensors 102, 104, 106, and 108. These sensors can include any type of sensor capable of obtaining wellness data, such as a biometric sensor, activity tracker, or the like. For example, sensors 102, 104, 106, and 108 can include, but are not limited to, a scale, blood pressure cuff, blood glucose monitor, electrocardiogram, step counter, gyroscope, accelerometer, SpO2 sensor, respiration sensor, posture sensor, stress sensor, photoplethysmogram, galvanic skin response sensor, temperature sensor, asthma inhaler, or the like. Sensors 102, 104, 106, and 108 can also include other types of sensors, such as audio sensors, ambient light sensors, electromagnetic sensors, touch sensors, capacitive sensors, and the like, for obtaining non-wellness data, such as situational data, temporal data, personal data, contact data, and the like data. In some examples, each sensor can be a separate device, while, in other examples, any combination of two or more of the sensors can be included within a single device. For example, the gyroscope, accelerometer, photoplethysmogram, galvanic skin response sensor, and temperature sensor can be included within a wearable electronic device, such as a smart watch, while the scale, blood pressure cuff, blood glucose monitor, Sp02 sensor, respiration sensor, posture sensor, stress sensor, and asthma inhaler can each be separate devices. While specific examples are provided, it should be appreciated that other sensors can be used and other combinations of sensors can be combined into a single device.

Sensors 102, 104, 106, and 108 can be used to measure wellness or non-wellness data continuously, intermittently, periodically, or at any other desired frequency or interval of time. For example, sensors 102, 104, 106, and 108 can be used to obtain a single measurement or multiple measurements over a length of time. Sensors 102, 104, 106, and 108 can be configured to measure wellness or non-wellness data at the same intervals of time, or can be configured to measure wellness or non-wellness data at different intervals of time. These intervals may be set by a user or may be a default setting for each sensor. Additionally, sensors 102, 104, 106, 108 can be used to measure wellness or non-wellness data at any time or location desired by the user. Moreover, sensors 102, 104, 106, and 108 can be used with or without the supervision of a healthcare provider. For example, a user can use sensors 102, 104, 106, and 108 to obtain sensor measurements at home without the supervision of a medical professional.

In some examples, user device 110 can include software sensor applications 113 (e.g., third party applications) associated with each of sensors 102, 104, 106, and 108 for interfacing with the sensors to allow user device 110 to receive the wellness or non-wellness data. In these examples, the applications 113 can use the device's APIs to store the wellness or non-wellness data in the wellness database 111 of user device 110. In some examples, the software sensor applications 113 can be Apps and device 110 can be a smart phone, tablet computer, or the like. It should be understood that "third party" can correspond to an entity different than the manufacturer of device 110 and/or the entity that created and/or maintains the operating system of device 110. In these instances, third party applications and their corresponding sensors can communicate and function within the operating system of device 110 according to a predefined device protocol associated with device 110.

The applications 113 can similarly use the device's APIs to access data stored in the wellness database 111. In other examples, user device 110 can be configured to share one or more communication formats with sensors 102, 104, 106, and 108 to allow user device 110 to receive and interpret the wellness or non-wellness data from the sensors. The received data can then be stored in the wellness database 111 of user device 110.

User device 110 can further receive wellness or non-wellness data from its own wellness or non-wellness data sensors 115, such as a GPS sensor, clock, gyroscope, accelerometer, or the like, from a user interacting with user device 110, from another entity, such as a physician, or from other non-sensor sources. For example, using the device's APIs, wellness or non-wellness data can be received from applications 117 on user device 110, such as a clock application, a calendaring application, a gaming application, an application from a healthcare provider, a messaging application, or the like. The wellness or non-wellness data from the applications 117 can originate from a user interacting with the applications, a remote database (e.g., database for a medical website), a healthcare provider institution (e.g., via the institution's App), or the like. In these examples, the usage of the application 117 (e.g., how long you play a video game application, when you play the video game, number of times interacting with a stock application, number of times interacting with a social networking application, length of time interacting with a social networking application, etc.), usage of user device 110 (e.g., length of time on the phone or number of text messages sent as determined from a phone payment application, time spent browsing the Internet as determined from the device's browser, etc.), time spent listening to music as determined from a music or streaming radio application, time spent using a remote application for controlling a television, amount of time or money spent on shopping websites, time spent on pornographic websites (e.g., to identify addictions), weather data from a weather application (e.g., to determine how weather affects a user's health), type of events occurring in the user's life as determined from a calendar (e.g., meetings, birthdays, holidays, etc.), interactions with certain people as determined from a contact list and/or calendar application and/or a messaging application and/or phone of user device 110, or the like, can be received by user device 110 and stored in the wellness database 111.

In some examples, default or user-selected settings can be provided to restrict the access that at least one application (e.g., at least one of applications 113 and 117) on user device 110 has to the wellness database 111 of user device 110 (for both storage and retrieval purposes) and to the sensor data generated by sensors 115 within user device 110 and/or sensor data generated by sensors 102, 104, 106, and 108. For example, an application for tracking a user's running sessions can be granted access to the data generated by the GPS sensor of user device 110, but can be prevented from accessing the user's blood pressure data stored in the wellness database 111. In some examples, an entity other than the owner of user device 110 can set the authorization settings for various applications on user device 110. For example, the manufacturer of user device 110 and/or the entity that created and/or maintains the operating system of user device 110 can evaluate the applications to determine if they should be given access to the user's wellness data and/or sensor data generated or received by user device 110. In some examples, these settings can be overridden by the user.

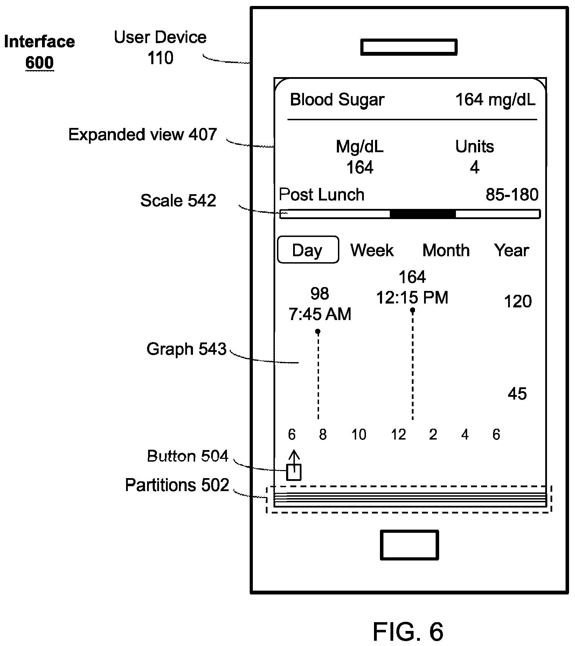

User device 110 can further include a display for displaying the stored wellness data or non-wellness data. A more detailed description of the interface of the display of user device 110 is described below with respect to FIGs. 6-17.

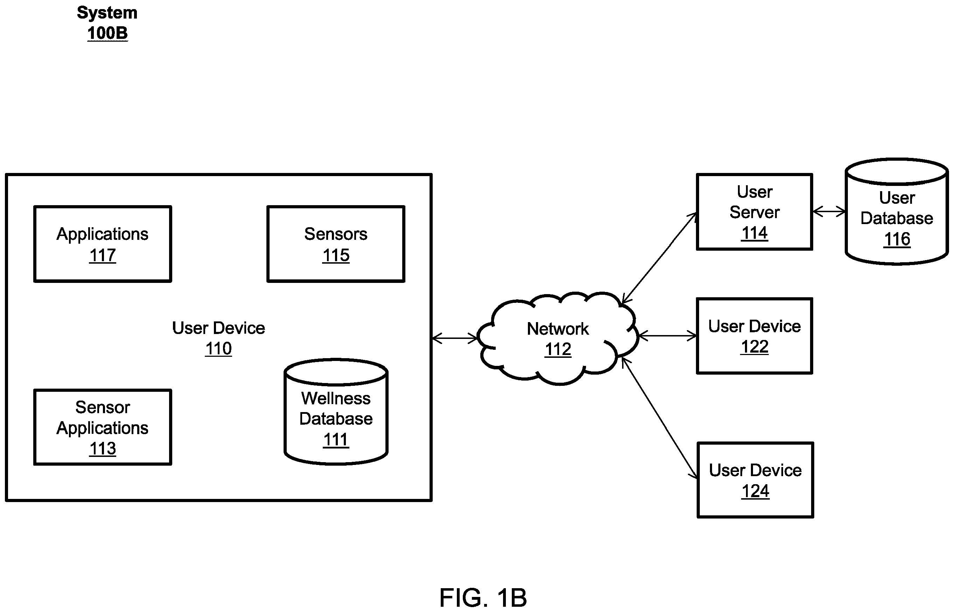

FIG. 1B illustrates system 100B for sharing user wellness data. Referring to FIG. 1B, user server 114 communicatively coupled to user device 110 via network 112, which can include the Internet, an intranet, or any other wired or wireless public or private network. User device 110 can be configured to securely transmit the aggregated wellness or non-wellness data and associated metadata stored on the device to user server 114 for storage in user database 116. In some examples, the wellness or non-wellness data and associated metadata can be transmitted to user server 114 for storage in user database 116 in response to an explicit request for such a transfer by the user of device 110, while, in other examples, the wellness or non-wellness data can be synced with the data in user database 116 continuously, periodically, intermittently, or at any desired frequency. In yet other examples, the user's wellness or non-wellness data can be stored only on user device 110 and may not be stored in an external database.

In some examples, user server 114 and user database 116 can be configured to securely store a user's wellness or non-wellness data using a public/private key system that only allows the owner of the wellness or non-wellness data to decrypt the data. Additionally, the wellness or non-wellness data stored in user database 116 can be stored anonymously (e.g., without identifying and/or personal information about the user, such as a legal name, username, time and location data, or the like). In this way, other users, hackers, and the owner/operator of user database 116 cannot determine the identity of the user associated with the data stored in database 116. In some examples, a user can access their wellness or non-wellness data stored in user database 116 from a user device that is different than the one used to upload the wellness or non-wellness data to user server 114. In these instances, the user can be required to provide login credentials to access their wellness or non-wellness data. User server 114 can be configured to perform the authorization process to restrict access to the data within user database 116.

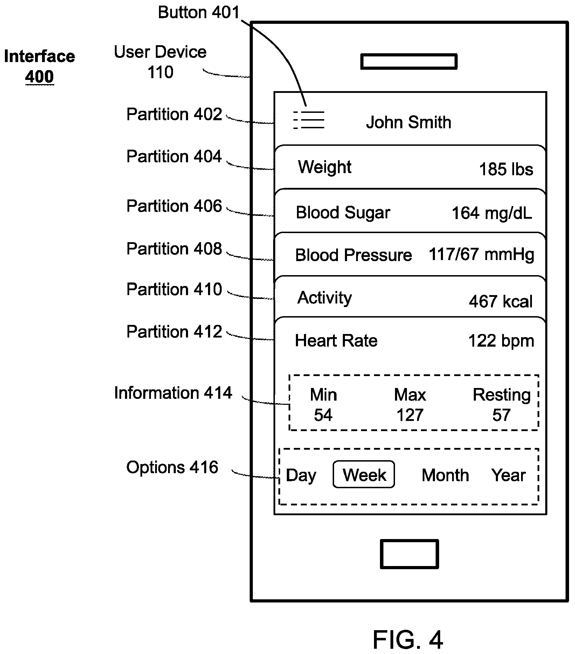

System 100B can further include any number of other user devices 122 and 124 coupled to network 112. In some examples, user devices 122 and 124 can be operated by the same user as user device 110. In these instances, the user can access their wellness or non-wellness data stored in user database 116 by providing user server 114 with the appropriate credentials. In some examples, wellness and non-wellness data can be synced between user database 116 and one or more of user device 110, 122, and 124. In other examples, the user of user devices 122 and 124 can be a person that is different than the user of user device 110. In these examples, the users of devices 122 and 124 cannot access the wellness or non-wellness data of the user of user device 110 without the authorization of the user of user device 110. If authorization is given, wellness or non-wellness data can be shared with the users of user devices 122 and 124. The sharing of this data will be discussed in greater detail below with respect to FIGs. 4-5.

In some examples, any of the above described sources of wellness or non-wellness data can be configured to measure, generate, or receive wellness or non-wellness data continuously, intermittently, periodically, or at any other desired frequency or interval of time. As such, the wellness or non-wellness data can similarly be stored or updated in wellness database 111 or user database 116 continuously, intermittently, periodically, or at any other desired frequency or interval of time. The frequencies and intervals of time used for measuring, generating, receiving, or storing wellness or non-wellness can be the same or they can be different. Additionally, these frequencies and intervals can be default values or they can be set by a user to provide the user with wellness or non-wellness data that has been updated within a desired length of time.

While not shown, it should be appreciated that many other user devices can be coupled to user server 114 through network 112 to collect and store wellness or non-wellness data for other users in a manner similar to that described above.

### USER DATABASE

As discussed above, a user's wellness or non-wellness data can be stored in user database 116 and can be shared with other users with the owning user's authorization. The other users can be any type of user, such as a friend, family member, caregiver, physician, social media provider, or the like. Different types and levels of authorization can be granted for the wellness or non-wellness data contained in wellness database 116.

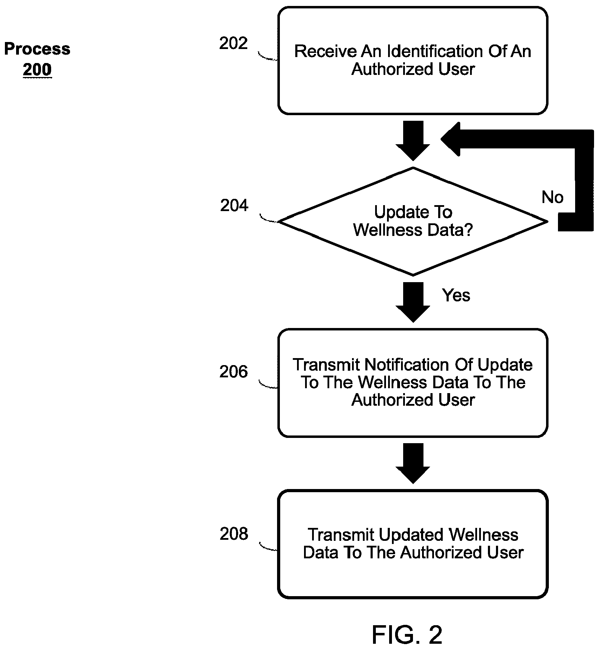

In some examples, the shared wellness or non-wellness data can be pushed to the user device (e.g., user device 122 or 124) of the authorized other user. FIG. 2 illustrates an example process 200 for authorizing and pushing wellness or non-wellness data to authorized other users. At block 202, an identification of an authorized other user can be received. This can be received by a user device (e.g., user device 110) from the user associated with the shared wellness or non-wellness data. The identification can include a username, legal name, contact information, or any other identifier or credential for the other user, along with a level of access, such as access to all of the wellness or non-wellness data or a subset of the wellness or non-wellness data. In some examples, the authorized other user can be grouped into categories of users (e.g., family, friends, other, etc.), where each category is associated with a particular set of wellness data types that those authorized other users are allowed to view. For example, users in the family category can be allowed to view all types of wellness data, while users in the friend category can only view activity data.

At block 204, the user's wellness or non-wellness data can be monitored by the user's device to determine if any updates to the data have been made. If no update has been made, the process can repeat block 204. If, however, an addition or change to the user's wellness or non-wellness data has been identified, the process can proceed to block 206. In some examples, any change to the user's wellness or non-wellness data can cause process 200 to proceed to block 206, while, in other examples, only changes to certain types of the user's wellness or non-wellness data can cause process 200 to proceed to block 206.

At block 206, a notification can be transmitted to one or more authorized other users. For example, a notification can be transmitted from the user's device (e.g., user device 110) to the device of the authorized other user (e.g., user device 122 or 124). The notification can include a message indicating that an update has been made to the user's wellness or non-wellness data. The process can then proceed to block 208.

At block 208, the updated wellness or non-wellness data can be transmitted to the authorized other user. For example, the updated wellness or non-wellness data can be transmitted from the user's device (e.g., user device 110) to the device of the authorized other user (e.g., user device 122 or 124). In some examples, this data can only be transmitted if the authorized other user indicated a desire to view the updated wellness or non-wellness data, while, in other examples, the updated wellness or non-wellness data can be transmitted to the user device of the authorized other user without receiving a request from the authorized other user to view the data.

The pushing of wellness or non-wellness data performed using process 200 can be useful in situations in which a user wants to keep a caregiver or family member (or other user) updated with his/her wellness or non-wellness data. For instance, an elderly parent can grant authorization to push his/her wellness or non-wellness data to a child to allow the child to easily monitor the elderly user's health or medication compliance without having to constantly request this information from the parent.

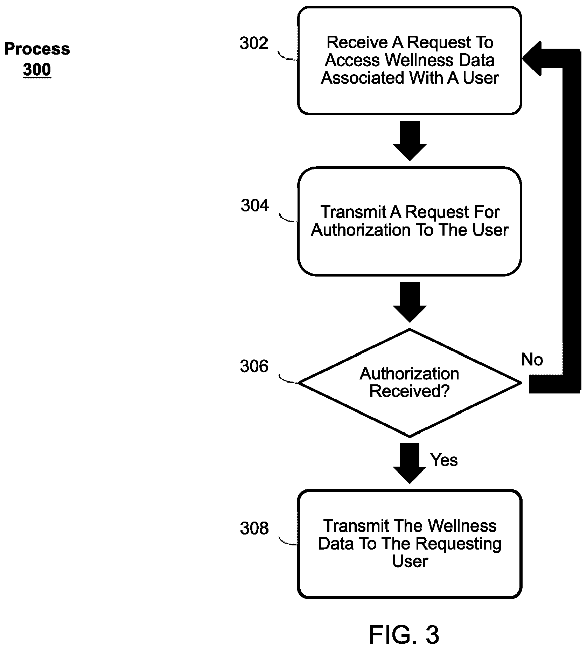

In other examples, a user's wellness or non-wellness data can be shared with other users by allowing other users to pull the wellness or non-wellness data. FIG. 3 illustrates an example process 300 for authorizing other users to pull wellness or non-wellness data. At block 302, a request to access a particular user's wellness or non-wellness data can be received. The request can identify the user associated with the requested wellness or non-wellness data and, optionally, the portion of the wellness or non-wellness data to be accessed. The identification can include a username, legal name, contact information, or any other identifier or credential for the user. The request can be received by a user device (e.g., user device 110) of the user associated with the requested data from a user device (e.g., user device 122 or 124) of the requesting user.

At block 304, a request for authorization can be transmitted to the user associated with the requested wellness or non-wellness data. In some examples, the request can be transmitted from the user device (e.g., user device 122 or 124) of the requesting user to the user device (e.g., user device 110) of the user associated with the requested data. The request can then be displayed to the user associated with the requested data by the user's device (e.g., user device 110).

At block 306, it can be determined if an authorization has been received from the user associated with the requested wellness or non-wellness data. The authorization can be received in any manner. For example, a prompt can be displayed to the user associated with the requested data on their user device 110. If authorization is denied, the process can return to block 302. If, however, authorization is granted, the process can proceed to block 308.

At block 308, the requested wellness or non-wellness data can be transmitted to the entity that requested access to the wellness or non-wellness data at block 302. The requested wellness or non-wellness data can be transmitted from the user's device (e.g., user device 110) to the requesting entity's device (e.g., user device 122 or 124).

In some examples, if authorization to access a user's wellness or non-wellness data is granted before a request is made at block 302, process 300 can proceed from block 302 to block 308 without performing blocks 304 or 306. For example, if a parent grants access to their wellness or non-wellness data to their child, a request by the child for the parent's wellness or non-wellness data can be received at block 302 and the requested wellness or non-wellness data can be transmitted to the child at block 308 without any intervening action by the parent.

The pulling of wellness or non-wellness data using process 300 can be useful in situations where an authorized other user does not want to be constantly updated with updates to a user's wellness or non-wellness data, but would like occasional access to the data. For example, a physician may want access to a patient's medical records and wellness or non-wellness data just prior to meeting with the patient. In this situation, the physician can request the patient's wellness or non-wellness data and can either receive the requested data or cause a request to be sent to the user device 110 of the patient. In response to receiving an authorization from the patient, the requested data can be sent from user device 110 to the physician's computing device (e.g., user device 122 or 124). In some examples, the received wellness or non-wellness data can be added to an electronic medical record (EMR) associated with the user.

In some examples, whether using a push or pull sharing model, the wellness or non-wellness data of a user may not be permanently stored on the user device of authorized other users. In this way, authorization to these other users can be revoked, causing the user's wellness data to be inaccessible by the formerly authorized other users. Additionally, in some examples, reports detailing or summarizing a user's wellness or non-wellness data can be generated. These reports can then be transmitted via email, secure transfer, or the like, to any desired recipient. In yet other examples, user device 110 can be configured to communicate some or all of the user's wellness or non-wellness data, such as medical insurance information, blood type, medical history, etc., to medical personnel using near field communication or another communication protocol. This can be useful when checking in to a hospital or during medical emergencies when relevant medical data is needed.

### AGGREGATED DATA VIEW

As discussed above, user device 110 can be configured to aggregate wellness or non-wellness data associated with a user that was received from the user, from one or more sensors 102, 104, 106, and 108, or from a non-sensor source. User device 110 can also be configured to display the user's aggregated wellness or non-wellness data in various ways using various interfaces.

FIG. 4 illustrates one example interface 400 that can be used by user device 110 to display a user's aggregated wellness or non-wellness data. Interface 400 can include any number of partitions 402, 404, 406, 408, 410, and 412, each containing a different type of wellness data associated with the user. In the aggregated view of FIG. 4, the partitions can be used to display a partial view of a portion of the different types of wellness data. In the examples shown in FIGs. 4-9, 11, and 13-14, the partitions are illustrated as having the appearance and associated animations of a stack of cards, where each card corresponds to a different partition (and thus, a different type of wellness data). It should be appreciated, however, that the partitions can be displayed in any other desired manner.

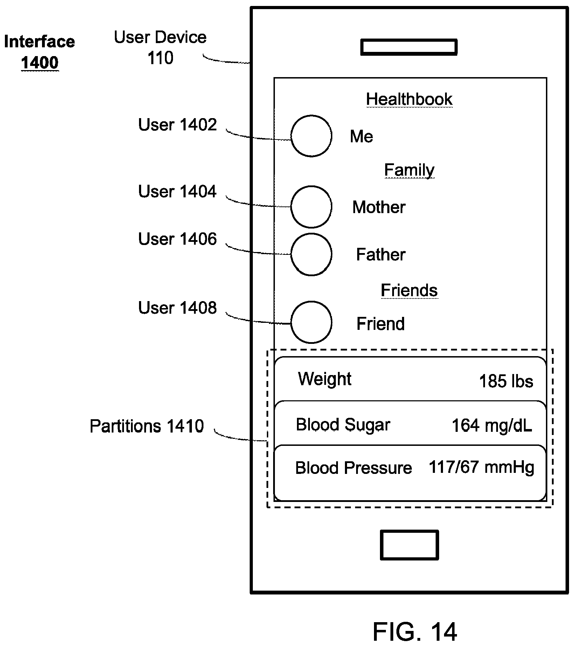

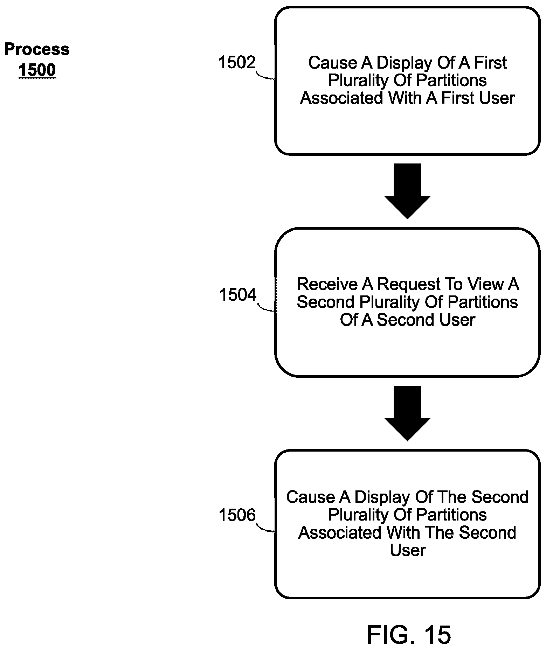

Interface 400 can include a first partition 402 displayed at the top of the interface that can include an identifier (e.g., name) of the user. As shown, the name "John Smith" of the user of user device 110 is displayed on partition 402. Interface 400 can further include button 401 for viewing wellness data associated with other users. This feature is described in greater detail below with respect to FIGs. 14 and 15.

Interface 400 can further include another partition 404 displayed adjacent to partition 402 such that it appears to be overlapping partition 402 like a stacked card. This partition can include an indication of the type of wellness data contained on the partition as well as a partial view (e.g., summary, example, or the like) of a portion the partition's wellness data. Specifically, partition 404 includes the word "Weight" to indicate that the partition contains weight data associated with the user "John Smith." Partition 404 can further include the most recent weight "185 lbs" of "John Smith."

Interface 400 can further include another partition 406 displayed adjacent to partition 404 such that it appears to be overlapping partition 404 like a stacked card. This partition can include an indication of the type of wellness data contained on the partition as well as a partial view (e.g., summary, example, or the like) of a portion the partition's wellness data. Specifically, partition 406 includes the words "Blood Sugar" to indicate that the partition contains blood sugar data associated with the user "John Smith." Partition 406 can further include the most recent blood sugar level "164 mg/dL" of "John Smith."

Interface 400 can further include another partition 408 displayed adjacent to partition 406 such that it appears to be overlapping partition 406 like a stacked card. This partition can include an indication of the type of wellness data contained on the partition as well as a partial view (e.g., summary, example, or the like) of a portion the partition's wellness data. Specifically, partition 408 includes the words "Blood Pressure" to indicate that the partition contains blood pressure data associated with the user "John Smith." Partition 408 can further include the most recent blood pressure measurement "117/67 mmHg" of "John Smith."

Interface 400 can further include another partition 410 displayed adjacent to partition 408 such that it appears to be overlapping partition 408 like a stacked card. This partition can include an indication of the type of wellness data contained on the partition as well as a partial view (e.g., summary, example, or the like) of a portion of the partition's wellness data. Specifically, partition 410 includes the word "Activity" to indicate that the partition contains activity data (e.g., calories burned) associated with the user "John Smith." Partition 410 can further include the current daily number of calories burned "467 kcal" by "John Smith."

Interface 400 can further include another partition 412 displayed adjacent to partition 410 such that it appears to be overlapping partition 410 like a stacked card. This partition can include an indication of the type of wellness data contained on the partition as well as a partial view (e.g., summary, example, or the like) of a portion of the partition's wellness data. Specifically, partition 412 includes the words "Heart Rate" to indicate that the partition contains heart rate data associated with the user "John Smith." Partition 412 can further include the most recent heart rate measurement "122 bpm" of "John Smith." Additionally, since partition 412 is at the top of the stack of partitions, additional information 414 and/or options 416 can be displayed on the face of the partition. In the illustrated example, the additional information includes the minimum, maximum, and resting heart rate of "John Smith" over the span of a week, which was selected using options 416.

The ordering of some or all of the partitions can be static or dynamic. For example, partition 402 containing the user's name can remain at the top of the display, while the remaining partitions can be ordered based on the time of day, how recently the wellness data associated with the partition is updated, the frequency that the wellness data associated with the partition is updated, whether a notable event is approaching (e.g., medication that should be taken, a sensor measurement that should be performed, etc.), or the like. The ordering can also be manually changed by the user. If interface 400 includes more partitions than can be displayed on a single screen, a vertical scrolling can be performed to display the remaining partitions. Alternatively, a 3D scrolling of the partitions may be performed to give the appearance of flipping through a stack of cards.

While specific example partition types have been provided above, it should be appreciated that partitions containing different types of wellness data can be included within interface 400. Additionally, users can generate customized partitions that can be used to display any desired type of user-entered data. For example, a partition can be created to track the medication compliance of a user. The user can enter the time, amount, and type of medicine taken and this data can be displayed on the created partition. Other custom partitions for different wellness data types, such as nutrition, sleep, smoking, or the like can also be generated.

Additionally, while specific examples of partial views (e.g., summary, example, or the like) of a portion of the partitions' wellness data have been provided above, it should be appreciated that the partial views can summarize or provide examples of the wellness data in different ways. For example, rather than display the most recent value of a particular wellness data type, a mean, mode, median, another single data point, or the like of the wellness data over all or a portion of time (e.g., a week, month, year, etc.) can be displayed on the partitions in the aggregated view. Alternatively, in other examples, a current and average value can be displayed on the partitions in the aggregated view. In yet other examples, the partitions in the aggregated view can include a display of a current value and a goal value for the type of wellness data.

The partitions 402, 404, 406, 408, 410, and 412 can be selected to display an expanded view of the partition containing reconfigured data, additional data, or an enlarged view of the original data contained on the partition. For example, a user can select a partition by tapping on the desired partition displayed on a touch sensitive display, can highlight and click on the partition using a mouse or other input device, or select the desired partition using any other input means. In response to a selection of a partition, user device 110 can hide the contents of the other partitions and display the selected partition on all or most of the display of user device 110.

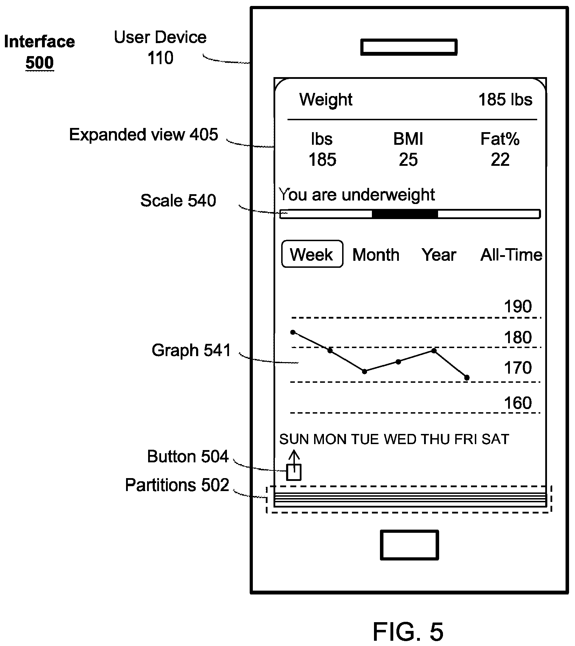

For example, FIG. 5 illustrates an example interface 500 that can be displayed in response to a user selecting partition 404 in interface 400. In interface 500, expanded view 405 of partition 404 can be displayed prominently within the display of user device 110, while the remaining partitions can be animated as being collapsed off the display into the stack of partitions 502 displayed at the bottom of the display. In the illustrated example, expanded view 405 can include additional weight-related data, such as the user's weight, body mass index (BMI), and fat percentage, that were not shown in interface 400. Expanded view 405 can further include summaries of the user's weight data, such as a sliding scale 540 indicating the user's weight relative to a range of weights and a graph 541 tracking the user's weight throughout the day, week, month, or year. Interface 500 can further include button 504 that can cause user device 110 to display options for sharing some or all of their wellness data using any desired communication medium, such as text message, email, social media provider, or the like. In these examples, the wellness data can be encrypted and sent from the user device 110 directly to the user device of the recipient (rather than from user server 114), where the wellness data can be decrypted. To return to the aggregated view of partitions shown in interface 400, the user can select (e.g., tap, click, or otherwise select) the stack of partitions 502. It should be appreciated that the contents of interface 500 and expanded view 405 are provided as examples, and that expanded view 405 can instead include any type or summary of weight-related data.

FIG. 6 illustrates an example interface 600 that can be displayed in response to a user selecting partition 406 in interface 400. In interface 600, expanded view 407 of partition 406 can be displayed prominently within the display of user device 110, while the remaining partitions can be animated as being collapsed off the display into the stack of partitions 502 displayed at the bottom of the display. In the illustrated example, expanded view 407 can include additional blood sugar-related data, such as the user's blood sugar and units that were not shown in interface 400. Expanded view 407 can further include summaries of the user's blood sugar data, such as a sliding scale 542 indicating the user's blood sugar after a meal relative to a range of typical blood sugar level and a graph 543 tracking the user's blood sugar throughout the day, week, month, or year. Interface 600 can also include button 504, described above. To return to the aggregated view of partitions shown in interface 400, the user can select (e.g., tap, click, or otherwise select) the stack of partitions 502. It should be appreciated that the contents of interface 600 and expanded view 407 are provided as examples, and that expanded view 407 can instead include any type or summary of blood sugar-related data.

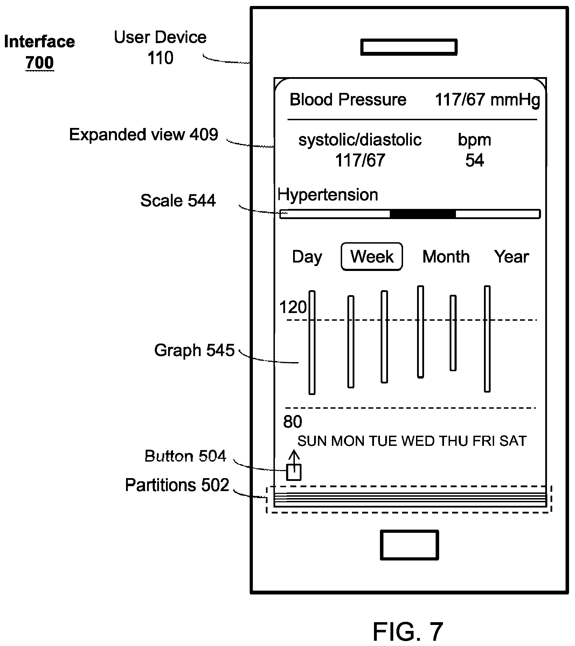

FIG. 7 illustrates an example interface 700 that can be displayed in response to a user selecting partition 408 in interface 400. In interface 700, expanded view 409 of partition 408 can be displayed prominently within the display of user device 110, while the remaining partitions can be animated as being collapsed off the display into the stack of partitions 502 displayed at the bottom of the display. In the illustrated example, expanded view 409 can include additional blood pressure-related data, such as the user's blood pressure and beats per minute (bpm) that were not shown in interface 400. Expanded view 409 can further include summaries of the user's blood pressure data, such as a sliding scale 544 indicating the user's blood pressure relative to typical blood pressure values and a graph 545 tracking the user's blood pressure throughout the day, week, month, or year. Interface 700 can also include button 504, described above. To return to the aggregated view of partitions shown in interface 400, the user can select (e.g., tap, click, or otherwise select) the stack of partitions 502. It should be appreciated that the contents of interface 700 and expanded view 409 are provided as examples, and that expanded view 409 can instead include any type or summary of blood pressure-related data.

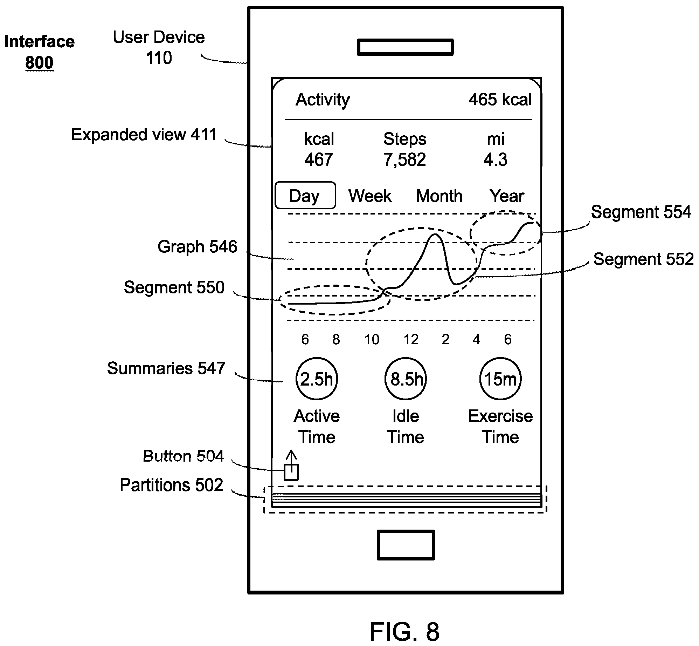

FIG. 8 illustrates an example interface 800 that can be displayed in response to a user selecting partition 410 in interface 400. In interface 800, expanded view 411 of partition 410 can be displayed prominently within the display of user device 110, while the remaining partitions can be animated as being collapsed off the display into the stack of partitions 502 displayed at the bottom of the display. In the illustrated example, expanded view 411 can include additional activity-related data, such as the calories burned, steps taken, and miles traveled by the user that were not shown in interface 400. Expanded view 411 can further include summaries of the user's blood pressure data, such as a graph 546 tracking the intensity of their activity throughout the day, week, month, or year, and summaries 547 showing the total time spent active, idle, and exercising. Interface 800 can also include button 504, described above. To return to the aggregated view of partitions shown in interface 400, the user can select (e.g., tap, click, or otherwise select) the stack of partitions 502. It should be appreciated that the contents of interface 800 and expanded view 411 are provided as examples, and that expanded view 411 can instead include any type or summary of activity-related data.

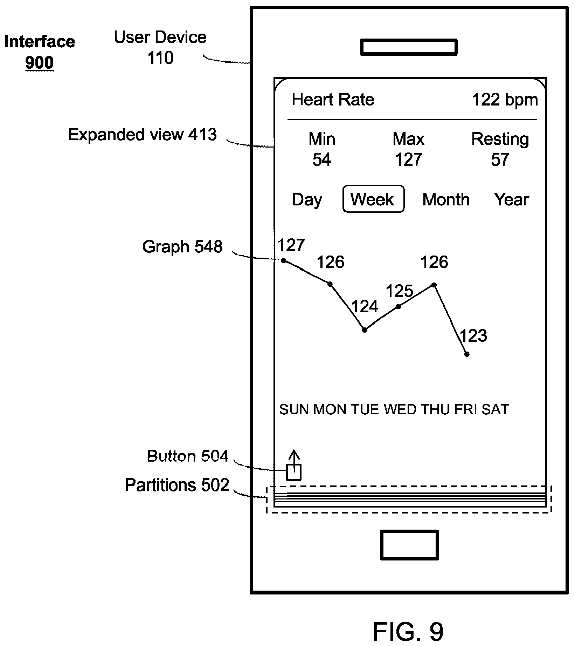

FIG. 9 illustrates an example interface 900 that can be displayed in response to a user selecting partition 412 in interface 400. In interface 900, expanded view 413 of partition 412 can be displayed prominently within the display of user device 110, while the remaining partitions can be animated as being collapsed off the display into the stack of partitions 502 displayed at the bottom of the display. In the illustrated example, expanded view 413 can include additional heart rate-related data, such as minimum, maximum, and resting heart rate of the user over a day, week, month, or year. However, unlike partitions 404, 406, 408, and 410, this data was previously displayed within interface 400 since partition 412 was displayed in the aggregated view as being located at the top of the stack of partitions. Expanded view 413 can further include a summary of the user's heart rate data, such as a graph 548 tracking the user's heart rate throughout the day, week, month, or year. Interface 900 can also include button 504, described above. To return to the aggregated view of partitions shown in interface 400, the user can select (e.g., tap, click, or otherwise select) the stack of partitions 502. It should be appreciated that the contents of interface 900 and expanded view 413 are provided as examples, and that expanded view 413 can instead include any type or summary of heart rate-related data.

In some examples, a user's wellness data used to generate the interfaces shown in FIGs. 4-9 may have been received from one or more sensor devices and/or applications. In these examples, the wellness data of the same type from different devices may be combined and displayed on a single partition. For example, the weight data shown in interface 500 in FIG. 5 can be obtained from repeated measurements taken using the same scale. Alternatively, some of the measurements could have been obtained from one scale, while the remaining measurements could have been obtained from one or more different scales. As a result, some points in graph 541 could have been generated using weight data from one scale, while other points in graph 541 could have been generated using weight data from one or more other scales. In another example, the activity data shown in interface 800 in FIG. 8 may have been obtained from a single step counting device. Alternatively, the data may be aggregated from a step counting device, a first GPS-enabled watch used to record a user's running session, and a second GPS-enabled watch used to record a user's rowing session. In these examples, graph 546 showing the intensity of the user's activity can be a segmented graph generated from a combination of the data obtained from the step tracking device and the first and second GPS-enabled watches, with each segment coming from one of the devices. In particular, graph 546 can include a first segment 550 generated from data from the step counting device, a second segment 552 generated from data from the first GPS-enabled watch, and a third segment 554 generated from data from the second GPS-enabled watch. Aggregating data in this way advantageously allows a user to record and view wellness data in a manner that does not directly tie the wellness data to a particular device or application (e.g., smartphone applications). While three segments are shown in FIG. 8, it should be appreciated that the segmented graph can include any number of segments corresponding to different sensors and/or applications.

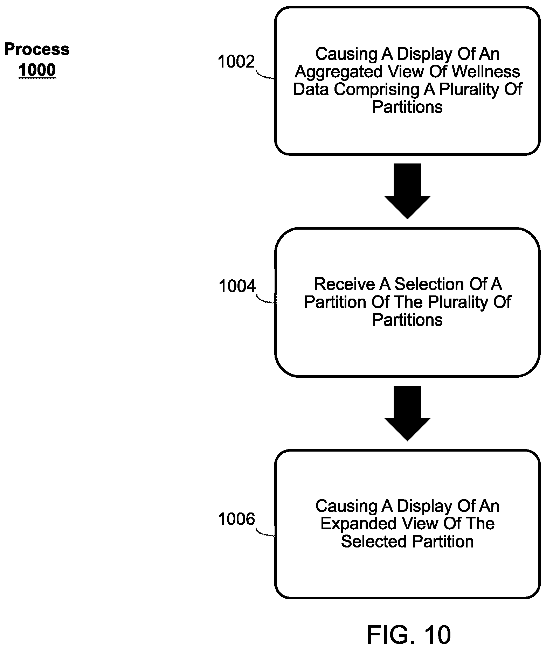

FIG. 10 illustrates an example process 1000 for displaying wellness data according to various examples. At block 1002, an aggregated view of wellness data containing a plurality of partitions similar or identical to partitions 402, 404, 406, 408, 410, and 412 can be displayed on a user device similar or identical to user device 110. The partitions can include an identifier for the type of wellness data displayed on the partition and a partial view (e.g., summary, example, or the like) of a portion of the type of wellness data associated with the partition. For example, an interface similar or identical to interface 400 can be displayed containing various wellness data partitions 402, 404, 406, 408, 410, and 412.

At block 1004, a selection of one of the displayed partitions can be received. The selection can be received by the user device, and can be in the form of a mouse click, tap on a touch sensitive display, or the like. In response to a selection of the partition, an expanded view of the selected partition can be displayed at block 1006. The expanded view can include a view showing a larger portion of the selected partition and at least one of reconfigured wellness data (e.g., a rearranged view of the wellness data displayed on the selected partition in the aggregated view displayed at block 1002), additional wellness data (e.g., wellness data that was not previously displayed on the selected partition in the aggregated view displayed at block 1002), or an enlarged view of the wellness data displayed in the aggregated view displayed at block 1002 (e.g., a larger view of the same wellness data displayed on the selected partition in the aggregated view displayed at block 1002). Additionally, partitions that were not selected can be reduced in size or removed from the display. For example, any of interfaces 500, 600, 700, 800, or 900 can be displayed in response to a selection of partition 404, 406, 408, 410, or 412 of interface 400, respectively.

In some examples, the display presented at block 1006 can include a selectable option (e.g., button 504) to share wellness data. In other examples, the display presented at block 1006 can further include a selectable option (e.g., partitions 502) to cause the aggregated view containing partitions displayed at block 1002 to again be displayed. The process can then return to block 1004, where a selection of another partition can be received.

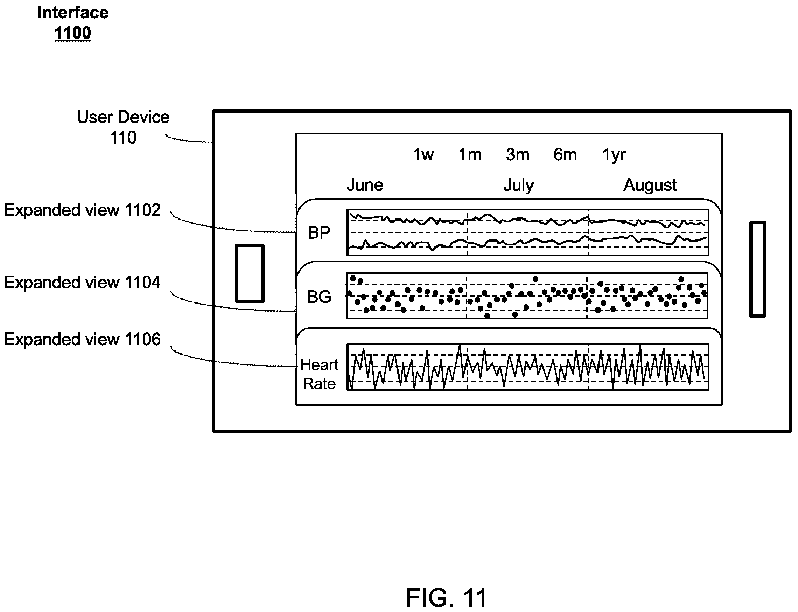

FIG. 11 illustrates an example interface 1100 that can be displayed in response to a user rotating user device 110 to a landscape view. In this orientation, user device 110 can display expanded views of a smaller subset of partitions than displayed in the aggregated view of interface 400. For example, interface 1100 can include expanded view 1102 of partition 408, expanded view 1104 of partition 406, and expanded view 1106 of partition 412. In the illustrated example, the expanded views of the partitions can include graph representations of the wellness data over various lengths of time. This can allow a user to view the displayed types of wellness data and see how they change relative to each other over time and to evaluate how a change in one type of wellness data relates to a change (or lack thereof) in another type of wellness data. For example, one type of wellness data that can be displayed can include medications taken over time. This data can be compared to a user's blood pressure and heart rate to determine if the medication is having a beneficial or detrimental effect. In some examples, user device 110 can select the types of wellness data to be displayed when user device 110 is rotated into landscape orientation. The selection can be made based on predetermined combinations of wellness data types that are often associated with each other or can be made based on an analysis of the various types of wellness data. In the latter example, wellness data types that are identified as potentially being correlated can be displayed. Wellness data types not shown in the initial display of interface 1100 can be viewed by scrolling through the partitions. Additionally, the partitions can be selected to display more detailed information in the same way as performed in interface 400.

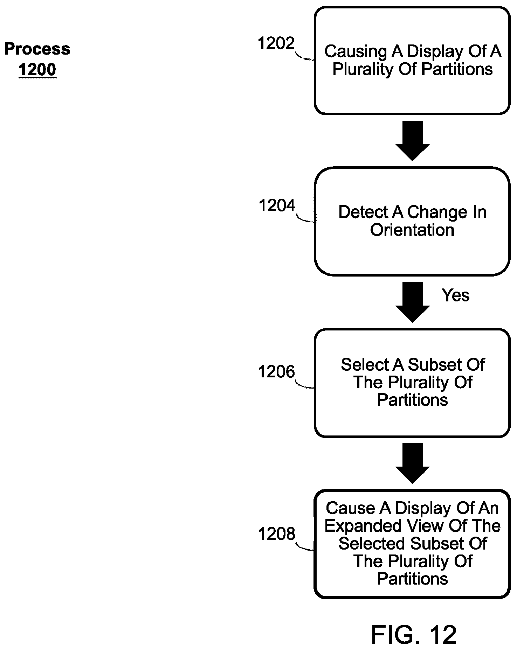

FIG. 12 illustrates an example process 1200 for displaying wellness data based on an orientation of the device according to various examples. At block 1202, an aggregated view containing a plurality of partitions similar or identical to partitions 402, 404, 406, 408, 410, and 412 can be displayed by a user device similar or identical to user device 110. The partitions can include an identifier for the type of wellness data displayed on the partition and a partial view (e.g., summary, example, or the like) of a portion the type of wellness data associated with the partition. For example, an interface similar or identical to interface 400 can be displayed containing various wellness data partitions 402, 404, 406, 408, 410, and 412.

At block 1204, a change in orientation of the user device can be detected. For example, a gyroscope and/or an accelerometer within the user device can indicate that the orientation of the device has been changed from portrait to landscape. In response to a detected change in orientation by a threshold amount, the process can proceed to block 1206.

At block 1206, a subset of the plurality of partitions displayed at block 1202 can be selected for display. In some examples, the subset can be a predetermined subset of the plurality of partitions. In other examples, the subset can be selected based on user-defined preferences for display. In yet other examples, the subset of the plurality of partitions can be selected based on an analysis of the wellness data sets associated with each partition to determine if any of the types of wellness data are potentially correlated. If a potential correlation is identified, the partitions associated with the potentially correlated data can be selected at block 1206.

At block 1208, expanded views of the subset of partitions selected at block 1206 can be displayed. In some examples, the identifier for the type of wellness data displayed on the expanded view of the partition and/or the summary, example, or other view of the type of wellness data associated with the partition can be different than that shown on the partitions at block 1202. For example, the summary or example data on each expanded view can be replaced with a graph representation of the wellness data over time, as shown in FIG. 11. In this way, relationships between the displayed types of wellness data can be observed. In some examples, in response to detecting a change in the orientation of the user device to a portrait orientation, the process can return to block 1202.

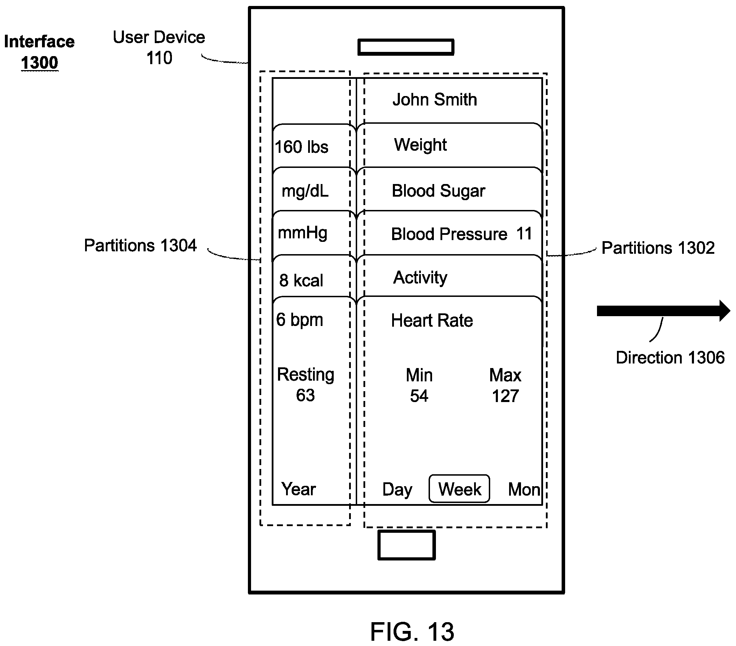

As discussed above, in some examples, the user of user device 110 can be granted access to the wellness data of other users. In these examples, user device 110 can allow the user to view the wellness data of other users in a similar format as discussed above with respect to FIGs. 4-12. For example, as shown by interface 1300 in FIG. 13, to view the wellness data of other users, the user can laterally scroll the contents of the interface in direction 1306 to cause a first set of partitions 1304 from a first user to be displayed in place of the second set of partitions 1302 from a second user. In particular, the second set of partitions 1302 for the second user can be translated off the display in the direction 1306 of the scroll, while the first plurality of partitions 1304 can be translated onto the display in the same direction 1306 but from the opposite side of the display. It should be appreciated that FIG. 13 illustrates a transitional state in which partitions 1302 are being removed from the display and partitions 1304 are being scrolled in direction 1306 onto the display. As such, only the left side of partitions 1302 and the right side of partitions 1304 are visible. As partitions 1302 and 1304 continue to be scrolled in direction 1306, a smaller portion of partitions 1302 may be visible while a larger portion of partitions 1304 may be visible until partitions 1302 are completely removed from the display and partitions 1304 occupy all or most of the display. While not shown, the top partition of partitions 1304 can include the first user's name and can be displayed as the screen continues to be scrolled in direction 1306. This scrolling operation can be performed any number of times (and in any direction) to scroll through the partitions for all users that the user of device 110 is authorized to access.

FIG. 14 illustrates another example interface 1400 for viewing the wellness data of other users. In some examples, interface 1400 can be displayed in response to a selection of button 401 of interface 400. As shown, interface 1400 can include a list of users 1402, 1404, 1406, and 1408 that have authorized the user of user device 110 to access their wellness data. In some examples, the circles next to each user's name can be replaced with an image associated with the user. These images can be the same image as used in device 110's contact list or can include another image. In the illustrated example, users 1402, 1404, 1406, and 1408 have been grouped into different categories (e.g., self, family, and friends). The categories can be used to logically group users together or, as discussed above, can indicate a level of authorization to view the wellness data of the other users. For example, users in the "family" category may have authorized a larger set of their wellness data to be viewed, while users in the "friend" category may have authorized a smaller set of their wellness data to be viewed. In response to a selection of one of users 1402, 1404, 1406, and 1408, partitions 1410 can be updated to reflect the wellness data of the selected user. This can include updating the types of wellness data displayed on the partitions (e.g., based on the types of wellness data the other user has authorized the user of user device 110 to view) as well as the associated partial views (e.g., summary, example, or the like) of portions the wellness data. In other examples, the list of users 1402, 1404, 1406, and 1408 can be displayed in response to vertically scrolling interface 400 up or down to expose the list of users.

FIG. 15 illustrates an exemplary process 1500 for displaying wellness data associated with two or more users according to various examples. At block 1502, a display of a first plurality of partitions associated with a first user can be displayed. The partitions can be similar or identical to partitions 402, 404, 406, 408, 410, and 412 and can be displayed by a user device similar or identical to user device 110. The partitions can include an identifier for the type of wellness data displayed on the partition and a partial view (e.g., summary, example, or the like) of a portion the type of wellness data associated with the partition. For example, an interface similar or identical to interface 400 can be displayed containing various wellness data partitions 402, 404, 406, 408,410, and 412.

At block 1504, a request to view a second plurality of partitions of a second user can be received. The second user can be a user that has authorized the first user to view their wellness data as described above. In some examples, the request can include a user input to scroll the display of the first plurality of partitions displayed at block 1502 and can be received by the user device in the form of rotation of a mouse wheel, an arrow key on a keyboard, a touch and/or swipe on a touch sensitive display, or the like. Similar to the example shown in FIG. 13, the scroll direction can be in a horizontal direction relative to the contents of the display and can be perpendicular to a scroll direction that causes additional partitions associated with the first user to be displayed. However, it should be appreciated that other scroll directions can be used. In other examples, the request to view the second plurality of partitions can be received in the form of a selection of the second user from a list of users. For example, the second user can be selected from a list of users similar to that shown in FIG. 14. The list can be displayed in response to a selection of a button (e.g., button 401) or other selection mechanism, such as vertically scrolling the first plurality of partitions displayed at block 1502 to expose the list of users. In response to receiving the request to view the second plurality of partitions at block 1504, the process can proceed to block 1506.

At block 1506, a display of the second plurality of partitions associated with the second user can be displayed. In some examples, when the request to view the second plurality of partitions received at block 1504 included a request to scroll the display of the first plurality of partitions, the first plurality of partitions can be translated off the display in the direction of the scroll, while the second plurality of partitions can be translated onto the display in the same direction but from the opposite side of the display, as shown in FIG. 13. In other examples, when the request to view the second plurality of partitions received at block 1504 included a selection of the second user from a list of users, the first plurality of partitions can be replaced with a display of the second plurality of partitions, as shown in FIG. 14. Additional requests to view wellness data partitions of other users can be received and blocks 1504 and 1506 can be repeated to display some or all of the sets of partitions associated with users that have authorized the user of device 110 to view their wellness data.

It should be appreciated that the processes described above can be combined. For example, process 1200 can be combined with process 1000 such that after the plurality of partitions are displayed at block 1002, a change in orientation of the user device can cause blocks 1204, 1206, and 1208 to be performed. Similarly, process 1500 can be combined with process 1000 such that after the plurality of partitions are displayed at block 1002, the receipt of a request to view partitions associated with other users can cause blocks 1504 and 1506 to be performed to display a plurality of partitions associated with another user.

As mentioned above, systems 100A and 100B can be configured to measure, generate, receive, or store wellness or non-wellness data continuously, intermittently, periodically, or at any other desired frequency or interval of time. Processes 1000, 1200, and 1500 can similarly be performed to update the display of wellness or non-wellness data continuously, intermittently, periodically, or at any other desired frequency or interval of time. For example, the device performing process 1000, 1200, or 1500 can receive updated wellness or non-wellness data from wellness database 111 or user database 116 continuously, intermittently, periodically, or at any other desired frequency or interval of time. In some examples, the device performing process 1000, 1200, or 1500 can receive updates to all wellness or non-wellness data at the same frequency or interval of time. In other examples, the device performing process 1000, 1200, or 1500 can be configured to receive updates to different types of data at different frequencies or intervals of time. For example, heart rate data can be updated every second, while weight data can be updated daily. These intervals and frequencies can be default values or can be selected by the user.

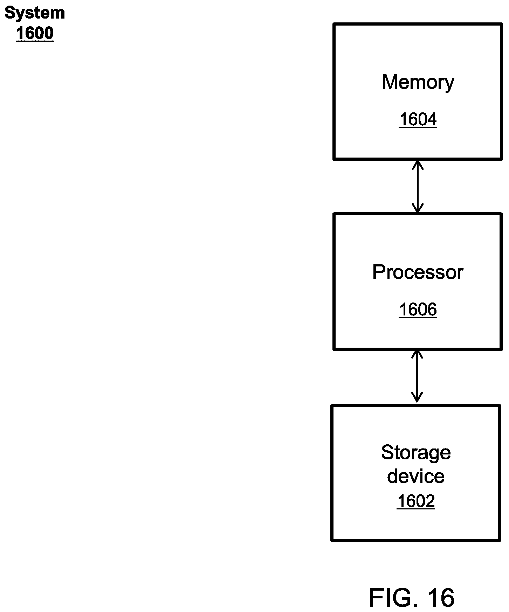

One or more of the functions relating to aggregating and sharing wellness data can be performed by a system similar or identical to system 1600 shown in FIG. 16. System 1600 can include instructions stored in a non-transitory computer readable storage medium, such as memory 1604 or storage device 1602, and executed by processor 1606. The instructions can also be stored and/or transported within any non-transitory computer readable storage medium for use by or in connection with an instruction execution system, apparatus, or device, such as a computer-based system, processor-containing system, or other system that can fetch the instructions from the instruction execution system, apparatus, or device and execute the instructions. In the context of this document, a "non-transitory computer readable storage medium" can be any medium that can contain or store the program for use by or in connection with the instruction execution system, apparatus, or device. The non-transitory computer readable storage medium can include, but is not limited to, an electronic, magnetic, optical, electromagnetic, infrared, or semiconductor system, apparatus or device, a portable computer diskette (magnetic), a random access memory (RAM), a read-only memory (ROM), an erasable programmable read-only memory (EPROM) (magnetic), a portable optical disc such a CD, CD-R, CD-RW, DVD, DVD-R, or DVD-RW, or flash memory such as compact flash cards, secured digital cards, USB memory devices, memory sticks, and the like.

The instructions can also be propagated within any transport medium for use by or in connection with an instruction execution system, apparatus, or device, such as a computer-based system, processor-containing system, or other system that can fetch the instructions from the instruction execution system, apparatus, or device and execute the instructions. In the context of this document, a "transport medium" can be any medium that can communicate, propagate or transport the program for use by or in connection with the instruction execution system, apparatus, or device. The transport medium can include, but is not limited to, an electronic, magnetic, optical, electromagnetic or infrared wired or wireless propagation medium.

In some examples, system 1600 can be included within user device 110 or user server 114. Processor 1606 can be configured to perform processes 200, 300, 1000, f0, and 1500. It is to be understood that the system is not limited to the components and configuration of FIG. 16, but can include other or additional components in multiple configurations according to various examples.

Although the disclosure and examples have been fully described with reference to the accompanying drawings, it is to be noted that various changes and modifications will become apparent to those skilled in the art. Such changes and modifications are to be understood as being included within the scope of the disclosure and examples as defined by the appended claims.

### NUMBERED STATEMENTS OF INVENTION

1. A computer-implemented method comprising:
   causing a display of an aggregated view of a plurality of types of wellness data, wherein the aggregated view comprises a plurality of partitions, each partition of the plurality of partitions associated with a type of the plurality of types of wellness data;
   receiving a selection of a partition of the plurality of partitions; and
   causing a display of an expanded view of the selected partition of the plurality of partitions.
2. The computer- implemented method of statement 1, wherein in the aggregated view, each partition of the plurality of partitions comprises an identifier of an associated type of wellness data and a first portion of the associated type of wellness data displayed therewith, and wherein the expanded view comprises a larger view of the selected partition of the plurality of partitions and a second portion of the associated type of wellness data displayed therewith.
3. The computer- implemented method of statement 2, wherein the first portion of the wellness data comprises a most recent value of the associated type of wellness data, and wherein the second portion of the associated type of wellness data comprises a representation of the associated type of wellness data over time.
4. The computer-implemented method of any of statements 1-3, wherein causing a display of the expanded view of the selected partition comprises causing a display of a collapsed set of partitions representing partitions of the plurality of partitions that were not selected.
5. The computer- implemented method of any of statements 1-4, wherein the plurality of partitions are ordered within the display based on a frequency of use of an associated type of wellness data, a time of most recently added value of the associated type of wellness data, or a time of day.
6. The computer- implemented method of any of statements 1-5, wherein the expanded view of the selected partition comprises a selectable element to share the wellness data associated with the partition through email or text message.
7. The computer- implemented method of any of statements 1-6, wherein the plurality of types of wellness data comprises weight data, blood sugar data, blood pressure data, activity data, or heart rate data.
8. The computer- implemented method of any of statements 1-7, wherein at least one of the plurality of types of wellness data is generated from sensor data obtained from a plurality of sensors.
9. The computer-implemented method of any of statements 1-8, wherein the expanded view comprises a graph of the associated type of wellness data, and wherein the graph comprises segments generated from wellness data obtained from different sensors.
10. The computer-implemented method of any of statements 1-9, wherein a partition of the plurality of partitions is a user-generated partition.
11. The computer- implemented method of any of statements 1-10, wherein the plurality of partitions have the appearance of a plurality of cards displayed in a stack.
12. A computer-implemented method comprising:
   causing, on a device, a display of a plurality of partitions, wherein each partition of the plurality of partitions is associated with a type of wellness data of a plurality of types of wellness data; in response to detecting a change in an orientation of the device, selecting a subset of the plurality of partitions; and
   causing a display of the selected subset of the plurality of partitions.
13. The computer- implemented method of statement 12, wherein the change in orientation of the device is detected based on data from at least one of a gyroscope of the device, an accelerometer of the device, or a combination thereof.
14. The computer-implemented method of any of statements 12-13, wherein detecting the change in the orientation of the device comprises detecting a threshold amount of change in the orientation of the device.
15. The computer- implemented method of any of statements 12-14, wherein each of the displayed subset of the plurality of partitions comprises a graph representation of at least a portion of the associated type of wellness data displayed therewith.
16. The computer- implemented method of any of statements 12-15, wherein selecting a subset of the plurality of partitions comprises:
   identifying correlations between the plurality of types of wellness data; and
   selecting partitions associated with correlated types of wellness data as the subset of the plurality of partitions.
17. A computer-implemented method comprising:
   causing a display of a first plurality of partitions associated with a first user, wherein each partition of the first plurality of partitions is associated with a type of wellness data of the first user; and
   in response to receiving a request to view a second plurality of partitions associated with a second user, causing a display of a second plurality of partitions associated with a second user, wherein each partition of the second plurality of partitions is associated with a type of wellness data of the second user.
18. The computer- implemented method of statement 17, wherein the request to view the second plurality of partitions comprises a request to scroll the displayed first plurality of partitions in a lateral direction.
19. The computer- implemented method of statement 17, wherein the request to view the second plurality of partitions comprises a selection of the second user from a list of users.
20. The computer-implemented method of any of statements 17-19, wherein the first user has been authorized by the second user to view the second plurality of partitions.
21. A computer-implemented method comprising:
   receiving an identification of a user authorized to access a set of wellness data;
   in response to detecting an update to the set of wellness data, transmitting a notification to the user authorized to access the set of wellness data notifying the user authorized to access the set of wellness data that the update to the set of wellness data has been detected; and
   transmitting at least a portion of the set of wellness data to the user authorized to access the set of wellness data.
22. The computer- implemented method of statement 21, wherein the identification of the user authorized to access the set of wellness data comprises a name, a username, or contact information.
23. The computer- implemented method of any of statements 21-22, wherein the at least a portion of the set of wellness data is transmitted in response to receiving a request from the user authorized to access the set of wellness data.
24. A computer-implemented method comprising:
   receiving, from a first user, a request to access wellness data associated with a second user;
   transmitting, to the second user, a request to authorize the first user to access the wellness data associated with the second user; and
   in response to receiving an authorization from the second user, transmitting the wellness data associated with the second user to the first user.
25. The computer- implemented method of statement 24, wherein the first user is a health care provider and the second user is a patient, and wherein the request to access the wellness data associated with the second user is received prior to an appointment between the first user and the second user.
26. The computer- implemented method of any of statements 24-25, wherein the request to authorize the first user to access the wellness data associated with the second user is displayed on a mobile device of the second user.
27. A non-transitory computer-readable storage medium comprising instructions for performing the method of any of statements 1-26.
28. A system comprising a processor capable of performing the method of any of statements 1-

## Claims

1. A method comprising:
at an electronic device of a first user comprising a display screen:
displaying, on the display screen, a first plurality of partitions associated with the first user, wherein each partition of the first plurality of partitions is associated with a type of wellness data of the first user, and wherein the first user has been authorized by a second user to access wellness data associated with the second user;
receiving the wellness data associated with the second user;
receiving a request to view a second plurality of partitions associated with the second user;
in response to receiving the request to view the second plurality of partitions associated with the second user, displaying, on the display screen, the second plurality of partitions associated with the second user, wherein each partition of the second plurality of partitions is associated with a type of wellness data of the second user; and
in accordance with a determination that the second user has revoked the authorization for the first user to access wellness data associated with the second user, causing the wellness data associated with the second user to be inaccessible.

2. The method of claim 1, wherein the request to view the second plurality of partitions comprises a request to scroll the displayed first plurality of partitions in a lateral direction.

3. The method of any of claims 1-2, wherein the request to view the second plurality of partitions comprises a selection of the second user from a list of users.

4. The method of any of claims 1-3, further comprising:
subsequent to displaying, on the display screen, the second plurality of partitions associated with the second user, receiving a request to view the first plurality of partitions associated with the first user; and
in response to receiving the request to view the first plurality of partitions associated with the first user, displaying, on the display screen, the first plurality of partitions associated with the first user.

5. A non-transitory computer-readable storage medium comprising instructions for performing the method of any of claims 1-4.

6. A system comprising a processor capable of performing the method of any of claims 1-4.
